# EUROPEAN PATENT SPECIFICATION

(11) **EP 2 025 664 B1**
(45) Date of publication and mention of the grant of the patent: **01.01.2014**
(21) Application number: 08252669.0
(22) Date of filing: 12.08.2008
(51) Int. Cl.: C07C 217/90, C07C 205/38, C08G 73/10

(54) **Aromatic diamine compound and aromatic dinitro**
Aromatische Diaminverbindung und aromatisches Dinitro
Composé diamine aromatique et dinitro aromatique

(30) Priority: 13.08.2007 JP 2007210900
(43) Date of publication of application: 18.02.2009
(73) Proprietor: Mitsubishi Gas Chemical Company, Inc., Tokyo (JP)
(72) Inventor: Uera, Kazuyoshi, Tokyo (JP); Ohno, Daisuke, Tokyo (JP); Ishii, Kenji, Tokyo (JP)
(74) Representative: McCluskie, Gail Wilson

(56) References cited:
- EP-A- 1 471 090
- WATANABE Y ET AL: "Synthesis and characterization of polyimides with low dielectric constants from aromatic dianhydrides and aromatic diamine containing phenylene ether unit" POLYMER, ELSEVIER SCIENCE PUBLISHERS B.V, GB, vol. 46, no. 16, 25 July 2005 (2005-07-25), pages 5903-5908, XP004971561 ISSN: 0032-3861

## Description

### Field of the Invention

The present invention relates to a novel aromatic diamine compound and a novel aromatic dinitro compound, each of which is obtained from a bifunctional phenylene ether oligomer having a specific structure as a raw material.

### Background of the Invention

Conventionally, aromatic diamine compounds are widely used as raw materials for functional high molecular weight materials such as bismaleimide, polyimide and thermosetting epoxy resins. In recent years, higher performance has been required in these fields so that higher physical properties have been more and more required as functional high molecular weight materials. As such physical properties, for example, heat resistance, weather resistance, chemical resistance, low water absorption properties, high fracture toughness, low dielectric constant, low dielectric loss tangent, moldability, flexibility, dispersibility in solvent and adhesive properties are required.

In the fields of information communications and calculators, for example, the signal band of information communication apparatus such as PHS and mobile phones and the CPU clock time of computers reach the GHz band. For inhibiting electric signals from damping because of insulators, a material having a small dielectric constant and a small dielectric loss tangent is desired for the insulators.

In the fields of printed wiring boards and semiconductor packages, for example, a temperature at soldering increases because of the recent introduction of lead-free solders. Therefore, high heat resistance, low water absorption properties, etc. are necessary to constituent materials of printed wiring boards, semiconductor packages or electronic parts for securing higher soldering reliability.

Further, the aromatic diamine compounds are used in the form of varnishes in these electronic material applications in most cases so that excellent solubility in solvent is desired in view of workability.

A variety of aromatic diamines and aromatic dinitro compounds, which are raw materials for the aromatic diamines, have been proposed for coping with these requirements. For example, aromatic diamines having fluorine atoms give high molecular weight materials having a low dielectric constant and a low dielectric loss tangent. However, the aromatic diamines having fluorine atoms have a problem about a decrease in heat resistance. It is known that aromatic diamines having fluorene skeleton give high molecular weight materials having a low dielectric constant and high heat resistance. However, a problem is that workability such as solubility in solvent is poor (for example, JP-A-10-152559).

It is thought that development of an aromatic diamine compound which has an oligomer structure and is excellent in low dielectric characteristics, heat resistance, low water absorption properties and solubility in solvent can cope with the above requirements about properties. However, such aromatic diamine having an oligomer structure has not been found yet.

Watanabe et al in Polymer, Elsevier Science Publishers B.V., GB, Polymer 46, No. 16, 25 July 2005, pages 5903 to 5908 disclose aromatic polyimides, including those of the following formula:

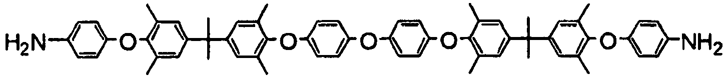

### Summary of the Invention

It is an object of the present invention to provide a novel aromatic diamine compound and a novel aromatic dinitro compound, each of which is a raw material used for obtaining a high molecular weight material having high heat resistance, a low dielectric constant, a low dielectric loss tangent and a low water absorption coefficient.

The present inventors have developed a bifunctional phenylene ether oligomer having a specific structure and having inherited excellent low dielectric characteristics and excellent heat resistance of a polyphenylene ether structure and a variety of derivatives thereof. The present inventors have made further diligent studies and as a result found that a terminal aromatic diamine compound can be obtained through a terminal aromatic dinitro compound from the bifunctional phenylene ether oligomer. On the basis of the above finding, the present inventors have completed the present invention.

According to the present invention, there is provided an aromatic diamine compound represented by the formula (1), 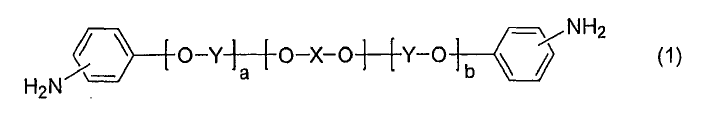 wherein -(O-X-O)- represents a moiety of the formula (2) or the formula (3), -(Y-O)- represents an arrangement of a moiety of the formula (4) or a random arrangement of at least two kinds of moieties of the formula (4), each of a and b is an integer of 0 to 100, provided that at least one of a and b is not 0, and each amino group is substituted at a para position or a meta position, 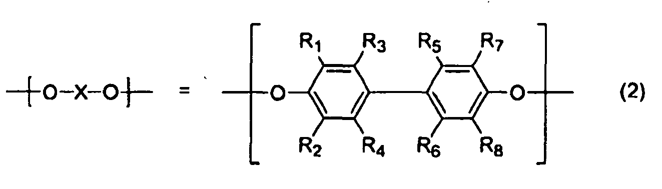 wherein R₁, R₂, R₃, R₇ and R₈ are the same or different and represent a halogen atom, an alkyl group having 6 or less carbon atoms or a phenyl group and R₄, R₅ and R₆ are the same or different and represent a hydrogen atom, a halogen atom, an alkyl group having 6 or less carbon atoms or a phenyl group, 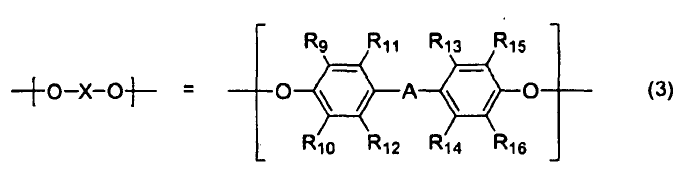 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 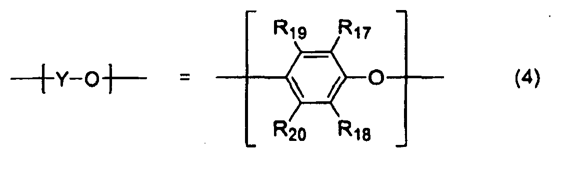 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

According to the present invention, there is further provided an aromatic dinitro compound represented by the formula (10), 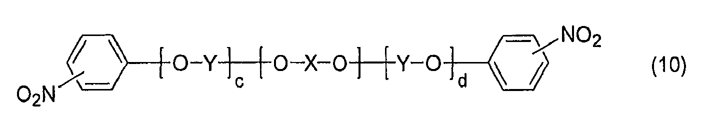 wherein -(O-X-O)- represents a moiety of the formula (11) or the formula (12), -(Y-O)- represents an arrangement of a moiety of the formula (13) or a random arrangement of at least two kinds of moieties of the formula (13), each of c and d is an integer of 0 to 100, provided that at least one of c and d is not 0, and each nitro group is substituted at a para position or a meta position, 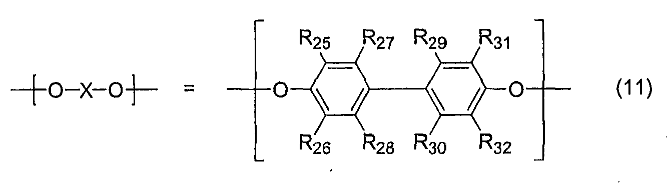 wherein R₂₅, R₂₆, R₂₇, R₃₁ and R₃₂ are the same or different and represent a halogen atom, an alkyl group having 6 or less carbon atoms or a phenyl group, R₂₈, R₂₉ and R₃₀ are the same or different and represent a hydrogen atom, a halogen atom, an alkyl group having 6 or less carbon atoms or a phenyl group, 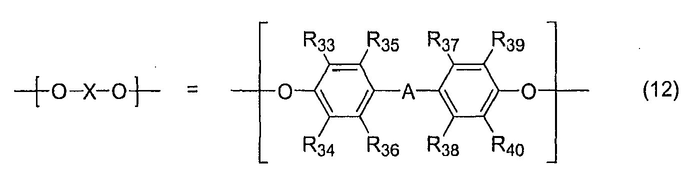 wherein R₃₃, R₃₄, R₃₅, R₃₆, R₃₇, R₃₈, R₃₉ and R₄₀ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 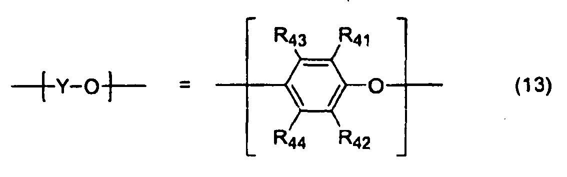 wherein R₄₁ and R₄₂ are the same or different and represent a halogen atom, an alkyl group having 6 or less carbon atoms or a phenyl group and R₄₃ and R₄₄ are the same or different and represent a hydrogen atom, a halogen atom, an alkyl group having 6 or less carbon atoms or a phenyl group.

According to the present invention, furthermore, there is provided a process for the production of the aromatic dinitro compound represented by the formula (10), comprising reacting a bifunctional phenylene ether oligomer obtained by oxidative coupling of a bifunctional phenol compound represented by the formula (19) or (20) and a monofunctional phenol compound represented by the formula (21) with a nitro halobenzene compound or a dinitro benzene compound, 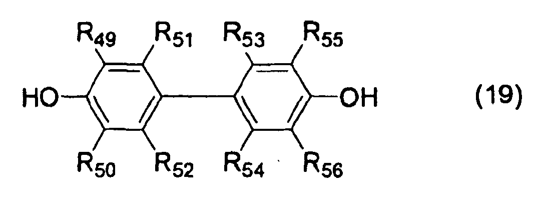 wherein R₄₉, R₅₀, R₅₁, R₅₅ and R₅₆ are the same or different and represent a halogen atom, an alkyl group having 6 or less carbon atoms or a phenyl group and R₅₂, R₅₃ and R₅₄ are the same or different and represent a hydrogen atom, a halogen atom, an alkyl group having 6 or less carbon atoms or a phenyl group, 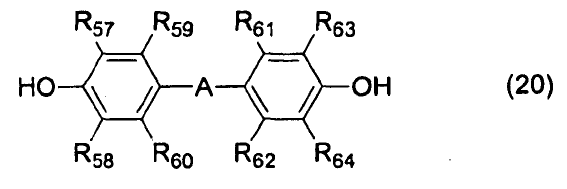 wherein R₅₇, R₅₈, R₅₉, R₆₀, R₆₁, R₆₂, R₆₃ and R₆₄ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 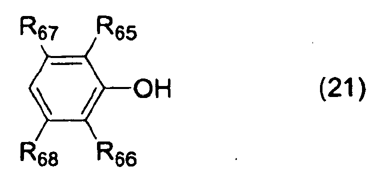 wherein R₆₅ and R₆₆ are the same or different and represent a halogen atom, an alkyl group having 6 or less carbon atoms or a phenyl group and R₆₇ and R₆₈ are the same or different and represent a hydrogen atom, a halogen atom, an alkyl group having 6 or less carbon atoms or a phenyl group.

### Brief Description of Drawings

Fig.1 shows IR spectrum of Resin "G" in Example 1.
Fig.2 shows ¹H NMR spectrum of Resin "G" in Example 1.
Fig. 3 shows FD mass spectrum of Resin "G" in Example 1.
Fig.4 shows IR spectrum of Resin "H" in Example 2.
Fig.5 shows ¹H NMR spectrum of Resin "H" in Example 2.
Fig. 6 shows FD mass spectrum of Resin "H" in Example 2.

### Effect of the Invention

The aromatic diamine compound provided by the present invention can be used as a raw material for bismaleimide, a raw material for polyimide, a curing agent for polyurethane, a curing agent for an epoxy resin, etc. The above aromatic diamine compound is remarkably useful as a raw material for a high-functional high molecular weight material having excellent heat resistance, low dielectric characteristics and low water absorption properties. Such high-functional high molecular weight material obtained therefrom can be used as a material having excellent electric characteristics and excellent moldability for wide uses such as an electrical insulating material, a molding material, a resin for a copper-clad laminate, a resin for a resist, a resin for sealing an electronic part, a resin for a color filter of liquid crystal, a coating, a variety of coating materials, an adhesive, a material for a buildup laminate, a resin for a flexible substrate, and a functional film.

The aromatic dinitro compound provided by the present invention can be easily transformed into the aromatic diamine compound, which is a raw material for a high molecular weight material having excellent properties as described above, by reducing nitro groups of the aromatic dinitro compound.

### Detailed Description of the Invention

The aromatic diamine compound provided by the present invention is represented by the formula (1). In the formula (1), -(O-X-O)- represents a moiety of the formula (2) wherein R₁, R₂, R₃, R₇ and R₈ are the same or different and represent a halogen atom, an alkyl group having 6 or less carbon atoms or a phenyl group and R₄, R₅ and R₆ are the same or different and represent a hydrogen atom, a halogen atom, an alkyl group having 6 or less carbon atoms or a phenyl group or a moiety of the formula (3) wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms. -(Y-O)- in the formula (1) represents an arrangement of a moiety of the formula (4) or a random arrangement of at least two kinds of moieties of the formula (4) wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group. Each of a and b in the formula (1) is an integer of 0 to 100, provided that at least one of a and b is not 0.

Examples of -A- in the formula (3) include bivalent organic groups such as methylene, ethylidene, 1-methylethylidene, 1,1-propylidene, 1,4-phenylenebis(1-methylethylidene), 1,3-phenylenebis(1-methylethylidene), cyclohexylidene, phenylmethylene, naphthyl methylene and 1-phenylethylidene. -A- in the formula (3) is not limited to these examples.

In the present invention, the aromatic diamine compound is preferably an aromatic diamine compound of the formula (1) wherein R₁, R₂, R₃, R₇, R₈, R₁₇ and R₁₈ represent an alkyl group having 3 or less carbon atoms, R₄, R₅, R₆, R₉, R₁₀, R₁₁, R₁₂, R₁₃, R₁₄, R₁₅, R₁₆, R₁₉ and R₂₀ represent a hydrogen atom or an alkyl group having 3 or less carbon atoms, more preferably an aromatic diamine compound of the formula (1) wherein -(O-X-O)- represented by the formula (2) or the formula (3) represents a moiety of the formula (5), the formula (6) or the formula (7) and -(Y-O)- represented by the formula (4) represents an arrangement of a moiety of the formula (8) or the formula (9) or a random arrangement of moieties of the formula (8) and the formula (9), 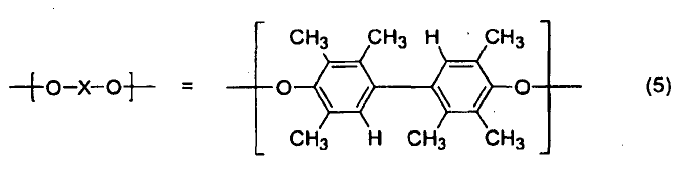 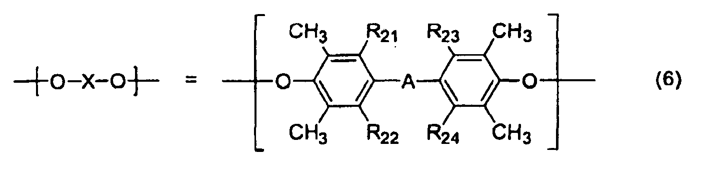 wherein R₂₁, R₂₂, R₂₃ and R₂₄ are the same or different and represent a hydrogen atom or a methyl group and -A-represents a linear, branched or cyclic bivalent hydrocarbon group having 20 or less carbon atoms, 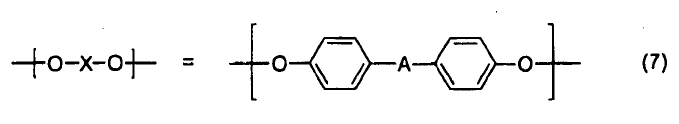 wherein -A- represents a linear, branched or cyclic bivalent hydrocarbon group having 20 or less carbon atoms.

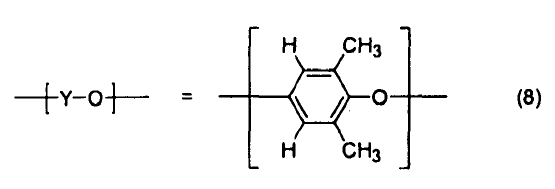

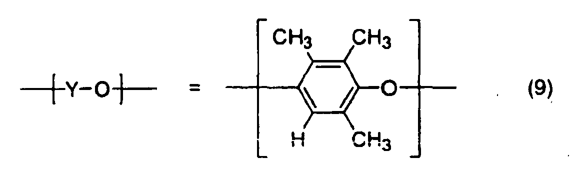

A process of producing the aromatic diamine compound provided by the present invention is not specially limited. The aromatic diamine compound of the present invention can be produced by any method. Preferably, it can be obtained by reducing an aromatic dinitro compound represented by the formula (10).

A method of the above-mentioned reduction is not specially limited. For example, it is possible to adopt a known method in which a nitro group is reduced to an amino group. The reduction reaction of the aromatic dinitro compound is, for example, carried out by reducing the aromatic dinitro compound to the aromatic diamine compound by use of hydrogen in a reaction solvent, which is inactive in the reaction, at a temperature of 20 to 200°C at a pressure of normal pressure to 50kgf/cm² in the presence of a hydrogenation catalyst such as a metal catalyst typified by nickel, palladium or platinum, a supported catalyst in which a metal like above is carried on a proper support, or a Raney catalyst of nickel, copper or the like. Examples of the above reaction solvent include aliphatic alcohols such as methanol, ethanol and isopropanol, ethylene glycol monoalkyl ethers such as methyl cellosolve and ethyl cellosolve, aromatic hydrocarbons such as toluene, benzene and xylene, and ethers such as tetrahydrofuran, dioxane, dipropyl ether, diethylene glycol dimethyl ether, diethylene glycol ethyl methyl ether and diethylene glycol diethyl ether. The reaction solvent is not limited to these examples so long as it is a solvent which dissolves the aromatic dinitro compound. The reaction solvent may be used singly or at least two reaction solvents may be used in combination.

The number average molecular weight of the aromatic diamine compound of the present invention is preferably in the range of from 500 to 3,000. When the number average molecular weight is less than 500, it is difficult to obtain electric characteristics that a phenylene ether structure has. When it exceeds 3,000, the reactivity of a terminal functional group decreases and the solubility into solvent also decreases.

The substitution position of an amino group of the aromatic diamine compound represented by the formula (1) is not specially limited so long as it is a para position or meth position.

Then, the aromatic dinitro compound of the present invention will be explained. The aromatic dinitro compound of the present invention is represented by the formula (10). In the formula (10), -(O-X-O)- represents a moiety of the formula (11) wherein R₂₅, R₂₆, R₂₇, R₃₁ and R₃₂ are the same or different and represent a halogen atom, an alkyl group having 6 or less carbon atoms or a phenyl group, R₂₈, R₂₉ and R₃₀ are the same or different and represent a hydrogen atom, a halogen atom, an alkyl group having 6 or less carbon atoms or a phenyl group, or a moiety of the formula (12) wherein R₃₃, R₃₄, R₃₅, R₃₆, R₃₇, R₃₈, R₃₉ and R₄₀ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms. In the formula (10), -(Y-O)- represents an arrangement of a moiety of the formula (13) or a random arrangement of at least two kinds of moieties of the formula (13) wherein R₄₁ and R₄₂ are the same or different and represent a halogen atom, an alkyl group having 6 or less carbon atoms or a phenyl group and R₄₃ and R₄₄ are the same or different and represent a hydrogen atom, a halogen atom, an alkyl group having 6 or less carbon atoms or a phenyl group. In the formula (10), each of c and d is an integer of 0 to 100, provided that at least one of c and d is not 0.

Examples of -A- in the formula (12) include bivalent organic groups such as methylene, ethylidene, 1-methylethylidene, 1,1-propylidene, 1,4-phenylenebis(1-methylethylidene), 1,3-phenylenebis(1-methylethylidene), cyclohexylidene, phenylmethylene, naphthyl methylene and 1-phenylethylidene. -A- is not limited to these examples.

In the present invention, the aromatic dinitro compound is preferably an aromatic dinitro compound of the formula (10) wherein R₂₅, R₂₆, R₂₇, R₃₁, R₃₂, R₄₁ and R₄₂ represent an alkyl group having 3 or less carbon atoms, R₂₈, R₂₉, R₃₀, R₃₃, R₃₄, R₃₅, R₃₆, R₃₇, R₃₈, R₃₉, R₄₀, R₄₃ and R₄₄ represent a hydrogen atom or an alkyl group having 3 or less carbon atoms, more preferably an aromatic dinitro compound of the formula (10) wherein - (O-X-O) - represented by the formula (11) or the formula (12) represents a moiety of the formula (14), the formula (15) or the formula (16) and -(Y-O)- represented by the formula (13) represents an arrangement of a moiety of the formula (17) or the formula (18) or a random arrangement of moieties of the formula (17) and the formula (18), 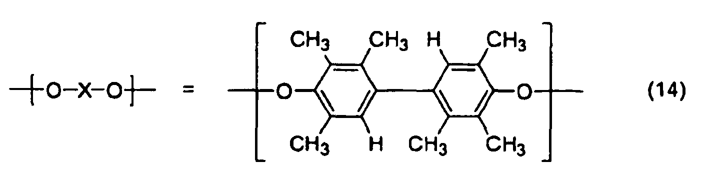 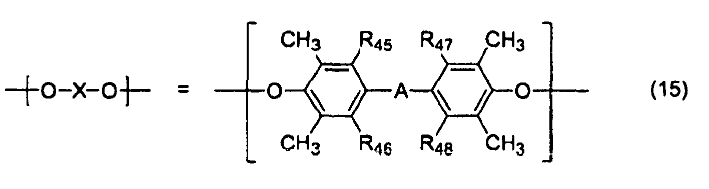 wherein R₄₅, R₄₆, R₄₇ and R₄₈ are the same or different and represent a hydrogen atom or a methyl group and -A-represents a linear, branched or cyclic bivalent hydrocarbon group having 20 or less carbon atoms, 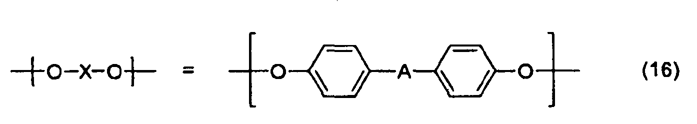 wherein -A- represents a linear, branched or cyclic bivalent hydrocarbon group having 20 or less carbon atoms.

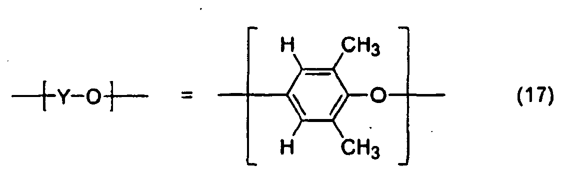

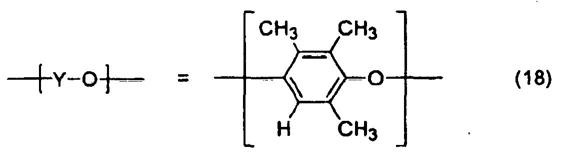

A process for producing the above aromatic dinitro compound represented by the formula (10) is not specially limited. The aromatic dinitro compound represented by the formula (10) can be produced by any method. Preferably, the aromatic dinitro compound represented by the formula (10) is produced by reacting a bifunctional phenylene ether oligomer, which is obtained by oxidative coupling of a bifunctional phenol compound and a monofunctional phenol compound, with a nitro halobenzene compound or a dinitro benzene compound in an organic solvent in the presence of a basic compound at a temperature of 50 to 250°C, more preferably 50 to 180°C, for 0.5 to 24 hours.

For example, the above bifunctional phenylene ether oligomer can be produced by dissolving a bifunctional phenol compound, a monofunctional phenol compound and a catalyst in a solvent and then introducing oxygen under heat with stirring. The bifunctional phenol compound is represented by the formula (19) or by the formula (20), and, preferably, R₄₉, R₅₀, R₅₁, R₅₅ and R₅₆ represent an alkyl group having 3 or less carbon atoms, R₅₂, R₅₃, R₅₄, R₅₇, R₅₈, R₅₉, R₆₀, R₆₁, R₆₂, R₆₃ and R₆₄, represent a hydrogen atom or an alkyl group having 3 or less carbon atoms, more preferably, R₄₉, R₅₀, R₅₁, R₅₄, R₅₅, R₅₆, R₅₇, R₅₈, R₆₃ and R₆₄ represent a methyl group, R₅₉, R₆₀, R₆₁ and R₆₂ represent a hydrogen atom or a methyl group, and R₅₂; R₅₃ represent a hydrogen group. Examples of the bifunctional phenol compound include 2,2'-,3,3'-,5,5'-hexamethyl-(1,1'-biphenyl)-4,4'- diol, 4,4'-methylenebis(2,6-dimethylphenol), 4,4'-dihydroxyphenyl methane and 4,4'-dihydroxy-2,2'- diphenylpropane. The bifunctional phenol compound is not limited to these examples. The monofunctional phenol compound is represented by the formula (21) and, preferably, R₆₅ and R₆₆ represent an alkyl group having 3 or less carbon atoms, R₆₇ and R₆₈ represent a hydrogen atom or an alkyl group having 3 or less carbon atoms, and, more preferably, R₆₅ and R₆₆ represent a methyl group, R₆₇ represents a hydrogen group or a methyl group, and R₆₈ represents a hydrogen group. The monofunctional phenol compound is typically 2,6-dimethylphenol or 2,3,6-trimethylphenol. The monofunctional phenol compound is not limited to these examples. The catalyst is, for example, a combination of a copper salt and an amine. Examples of the copper salt include CuCl, CuBr, CuI, CuCl₂ and CuBr₂. Examples of the amine include di-n-butylamine, n-butyldimethylamine, N,N'-di-t-butylethylenediamine, pyridine, N,N,N'N'-tetramethylethylenediamine, piperidine and imidazole. The catalyst is not limited to these examples. Examples of the solvent include toluene, methanol, methyl ethyl ketone and xylene. The solvent is not limited to these examples.

Specific examples of the aforesaid nitro halobenzene compound include 4-chloronitrobenzene, 3-chloronitrobenzene, 2-chloro-4-nitrotoluene, 2-chloro-5-nitrotoluene, 2-chloro-6-nitrotoluene, 3-chloro-5-nitrotoluene, 3-chloro-6-nitrotoluene, 4-chloro-2-nitrotoluene, 4-fluoronitrobenzene, 3-fluoronitrobenzene, 2-fluoro-4-nitrotoluene, 2-fluoro-5-nitrotoluene, 2-fluoro-6-nitrotoluene, 3-fluoro-5-nitrotoluene, 3-fluoro-6-nitrotoluene and 4-fluoro-2-nitrotoluene. Specific examples of the aforesaid dinitro benzene compound include 1,3-dinitrobenzene, 1,4-dinitrobenzene, 4-methyl-1,3-dinitrobenzene, 5-methyl-1,3-dinitrobenzene and2-methyl-1,4-dinitrobenzene. For obtaining an aromatic dinitro compound having nitro groups substituted at para positions, 4-chloronitrobenzene is preferred. For obtaining an aromatic dinitro compound having nitro groups substituted at meth positions, 1,3-dinitrobenzene is preferred.

Preferable examples of the aforesaid organic solvent include aromatic hydrocarbons such as benzene, toluene and xylene, ketones such as acetone and methyl ethyl ketone, halogenated hydrocarbons such as 1,2-dichloroethane and chlorobenzene, ethers such as 1,2-dimethoxyethane, diethylene glycol dimethyl ether, diethylene glycol ethyl methyl ether, diethylene glycol diethyl ether, tetrahydrofuran, 1,3-dioxane and 1,4-dioxane and non-protonic polar solvents such as N,N-dimethylformamide, N,N-dimethylacetamide, dimethylsulfoxide, N-methyl-2-pyrrolidone and sulfolane. The organic solvent is not limited to these examples so long as it is a solvent which dissolves the bifunctional phenylene ether oligomer and the nitro halobenzene compound or the dinitro benzene compound. The organic solvent can be used singly or at least two organic solvents can be used in combination. Examples of the aforesaid basic compound include a hydroxide of an alkali metal, a hydrogen carbonate of an alkali metal, a carbonate of an alkali metal and an alkoxide compound of an alkali metal. The basic compound can be used singly or at least two basic compounds can be used in combination.

The number average molecular weight of the aromatic dinitro compound of the present invention is preferably in the range of 500 to 3, 000. When the number average molecular weight is less than 500, it is difficult to obtain electric characteristics that a phenylene ether structure has. When it exceeds 3,000, the reactivity of a terminal functional group decreases and the solubility into solvent also decreases.

The substitution position of a nitro group of the aromatic dinitro compound represented by the formula (10) is not specially limited so long as it is a para position or meth position.

The thus-obtained aromatic diamine compound and aromatic dinitro compound of the present invention can be suitably used as a raw material for bismaleimide or polyimide (polyetherimide) or as a curing agent for polyurethane or epoxy resins.

Examples

The present invention will be more concretely explained with reference to Examples hereinafter, while the present invention shall not be specially limited to these Examples. A number average molecular weight and a weight average molecular weight were obtained by a gel permeation chromatography (GPC) method (calculated as polystyrene). Tetrahydrofuran (THF) was used for a developing solvent of GPC. A hydroxyl group equivalent was obtained by quantification of a terminal hydroxyl group by means of titration.

### Synthetic Example 1

### (Synthesis of bifunctional phenylene ether oligomer)

A longitudinally long reactor having a volume of 12 liters and equipped with a stirrer, a thermometer, an air-introducing tube and baffleplates was charged with 3.88 g (17.4 mmol) of CuBr₂, 0.75 g (4.4 mmol) of N,N'-di-t-butylethylenediamine, 28.04 g (277.6 mmol) of n-butyldimethylamine and 2,600 g of toluene. The mixture was stirred at a reaction temperature of 40 °C. Separately, 129.32 g (0.48 mol) of 2,2',3,3',5,5'-hexamethyl-(1,1'-biphenyl) -4,4'-diol, 292.19 g (2.40 mol) of 2,6-dimethylphenol, 0.51 g (2.9 mmol) of N, N' -di-t-butylethylenediamine and 10.90 g (108.0 mmol) of n-butyldimethylamine were dissolved in 2,300 g of methanol, to obtain a mixed solution. The mixed solution was dropwise added to the mixture in the reactor over 230 minutes with stirring. During the above addition of the mixed solution, bubbling was continuously carried out with a nitrogen-air mixed gas having an oxygen concentration of 8 % at a flow velocity of 5.2L/min. After the completion of the addition, 1,500 g of water in which 19.89 g (52.3 mmol) of tetrasodium ethylenediamine tetraacetate was dissolved was added to the stirred mixture to terminate the reaction. An aqueous layer and an organic layer were separated. Then, the organic layer was washed with 1N hydrochloric acid aqueous solution and then washed with pure water. The thus-obtained solution was concentrated to 50 wt% with an evaporator, to obtain 833.40 g of a toluene solution of a bifunctional phenylene ether oligomer (resin "A") . The resin "A" had a number average molecular weight of 930, a weight average molecular weight of 1, 460 and a hydroxyl group equivalent of 465.

### Synthetic Example 2

### (Synthesis of bifunctional phenylene ether oligomer)

A longitudinally long reactor having a volume of 12 liters and equipped with a stirrer, a thermometer, an air-introducing tube and baffleplates was charged with 9.36 g (42.1 mmol) of CuBr₂, 1.81 g (10.5 mmol) of N,N'-di-t-butylethylenediamine, 67.77 g (671.0 mmol) of n-butyldimethylamine and 2,600 g of toluene. The mixture was stirred at a reaction temperature of 40 °C. Separately, 129.32 g (0.48 mol) of 2,2',3,3',5,5'-hexamethyl-(1,1'-biphenyl) -4,4'-diol, 878.4g (7.2mol) of 2,6-dimethylphenol, 1. 22 g (7.2 mmol) of N,N'-di-t-butylethylenediamine and 26.35 g (260.9 mmol) of n-butyldimethylamine were dissolved in 2,300 g of methanol, to obtain a mixed solution. The mixed solution was dropwise added to the mixture in the reactor over 230 minutes with stirring. During the above addition of the mixed solution, bubbling was continuously carried out with a nitrogen-air mixed gas having an oxygen concentration of 8 % at a flow velocity of 5.2L/min. After the completion of the addition, 1,500 g of water in which 48.06 g (126.4 mmol) of tetrasodium ethylenediamine tetraacetate was dissolved was added to the stirred mixture to terminate the reaction. An aqueous layer and an organic layer were separated. Then, the organic layer was washed with 1N hydrochloric acid aqueous solution and then washed with pure water. The thus-obtained solution was concentrated to 50 wt% with an evaporator, to obtain 1,981 g of a toluene solution of a bifunctional phenylene ether oligomer (resin "B") . The resin "B" had a number average molecular weight of 1,975, a weight average molecular weight of 3,514 and a hydroxyl group equivalent of 990.

### Synthetic Example 3

### (Synthesis of bifunctional phenylene ether oligomer)

A longitudinally long reactor having a volume of 12 liters and equipped with a stirrer, a thermometer, an air-introducing tube and baffleplates was charged with 13.1 g (0.12 mol) of CuCl, 707.0 g (5.5 mol) of di-n-butylamine and 4,000 g of methyl ethyl ketone. The mixture was stirred at a reaction temperature of 40 °C. A solution of 410.2 g (1.6 mol) of 4,4' -methylenebis (2, 6-dimethylphenol) and 586.5 g (4.8 mol) of 2,6-dimethylphenol in 8,000 g of methyl ethyl ketone was dropwise added to the mixture in the reactor over 120 minutes with stirring. During the above addition of the solution, bubbling was continuously carried out with 2 L/min of air. A disodium dihydrogen ethylenediamine tetraacetate aqueous solution was added the stirred mixture to terminate the reaction. Then, washing was three times carried out with 1N hydrochloric acid aqueous solution and then washing was carried out with ion-exchanged water. The thus-obtained solution was concentrated with an evaporator and then dried under a reduced pressure, to obtain 946.6 g of a bifunctional phenylene ether oligomer (resin "C"). The resin "C" had a number average molecular weight of 801, a weight average molecular weight of 1,081 and a hydroxyl group equivalent of 455.

### Synthetic Example 4

### (Synthesis of bifunctional phenylene ether oligomer)

A longitudinally long reactor having a volume of 12 liters and equipped with a stirrer, a thermometer, an air-introducing tube and baffleplates was charged with 13.1 g (0.12 mol) of CuCl, 707.0 g (5.5 mol) of di-n-butylamine and 4,000 g of methyl ethyl ketone. The mixture was stirred at a reaction temperature of 40 °C. A solution of 82.1 g (0.32 mol) of 4,4'-methylenebis (2, 6-dimethylphenol) and 586.5 g (4.8 mol) of 2,6-dimethylphenol in 8,000 g of methyl ethyl ketone was dropwise added to the mixture in the reactor over 120 minutes with stirring. During the above addition of the solution, bubbling was continuously carried out with 2 L/min of air. A disodium dihydrogen ethylenediamine tetraacetate aqueous solution was added to the stirred mixture, to terminate the reaction. Then, washing was three times carried out with 1N hydrochloric acid aqueous solution and then washing was carried out with ion-exchanged water. The thus-obtained solution was concentrated with an evaporator and then dried under a reduced pressure, to obtain 632.5 g of a bifunctional phenylene ether oligomer (resin "D"). The resin "D" had a number average molecular weight of 1,884, a weight average molecular weight of 3,763 and a hydroxyl group equivalent of 840.

### Synthetic Example 5

### (Synthesis of bifunctional phenylene ether oligomer)

A longitudinally long reactor having a volume of 2 liters and equipped with a stirrer, a thermometer, an air-introducing tube and baffleplates was charged with 18.0 g (78.8 mmol) of 4,4'-dihydroxy-2,2'-diphenylpropane(bisphenol A), 0.172 g (0.77 mmol) of CuBr₂, 0.199 g(1.15 mmol) of N,N'-di-t-butylethylenediamine, 2.10 g (2.07 mmol) of n-butyldimethylamine, 139 g of methanol and 279 g of toluene. Separately, 48.17 g (0.394 mol) of 2,6-dimethylphenol, 0.245 g(1.44 mmol) of N,N'-di-t-butylethylenediamine and 2.628 g (25.9 mmol) of n-butyldimethylamine were dissolved in 133 g of methanol and 266 g of toluene, to obtain a mixed solution. The mixed solution was dropwise added to the reactor, in which the mixture was stirred at a liquid temperature of 40°C, over 132 minutes. During the above addition of the mixed solution, bubbling was continuously carried with air at a flow velocity of 0.5 L/min. After the completion of the addition of the mixed solution, the resultant mixture was further stirred for 120 minutes. Then, 400 g of water in which 2.40 g of tetrasodium ethylenediamine tetraacetate was dissolved was added to the stirred mixture to terminate the reaction. An aqueous layer and an organic layer were separated. Then, washing with pure water was carried out. The thus-obtained solution was concentrated with an evaporator. The concentrated solution was dried in vacuum at 120°C for 3 hours, to obtain 54.8 g of a bifunctional phenylene ether oligomer (resin "E"). The resin "E" had a number average molecular weight of 1,348, a weight average molecular weight of 3,267 and a hydroxyl group equivalent of 503.

### Synthetic Example 6

### (Synthesis of bifunctional phenylene ether oligomer)

A longitudinally long reactor having a volume of 12 liters and equipped with a stirrer, a thermometer, an air-introducing tube and baffleplates was charged with 3.88 g (17.4 mmol) of CuBr₂, 0.75 g (4.4 mmol) of N,N'-di-t-butylethylenediamine, 28.04 g (277.6 mmol) of n-butyldimethylamine and 2,600 g of toluene. The mixture was stirred at a reaction temperature of 40 °C. Separately, 129.3 g (0.48 mol) of 2,2',3,3',5,5'-hexamethyl-(1,1'-biphenyl) -4,4'-diol, 233.7 g (1.92 mol) of 2,6-dimethylphenol, 64.9 g (0.48 mol) of 2,3,6-trimethylphenol, 0.51 g (2.9 mmol) of N,N'-di-t-butylethylenediamine and 10.90 g (108.0 mmol) of n-butyldimethylamine were dissolved in 2, 300 g of methanol, to obtain a mixed solution. The mixed solution was dropwise added to the mixture in the reactor over 230 minutes with stirring. During the above addition of the mixed solution, bubbling was continuously carried out with a nitrogen-air mixed gas having an oxygen concentration of 8 % at a flow velocity of 5.2L/min. After the completion of the addition, 1,500 g of water in which 19.89g (52.3 mmol) of tetrasodium ethylenediamine tetraacetate was dissolved was added to the stirred mixture to terminate the reaction. An aqueous layer and an organic layer were separated. The organic layer was washed with 1N hydrochloric acid aqueous solution and then washed with pure water. The thus-obtained solution was concentrated to 50 wt% with an evaporator, to obtain 836. 5 g of a toluene solution of a bifunctional phenylene ether oligomer (resin "F") . The resin "F" had a number average molecular weight of 986, a weight average molecular weight of 1,530 and a hydroxyl group equivalent of 471.

### Example 1

### (Synthesis of aromatic dinitro compound)

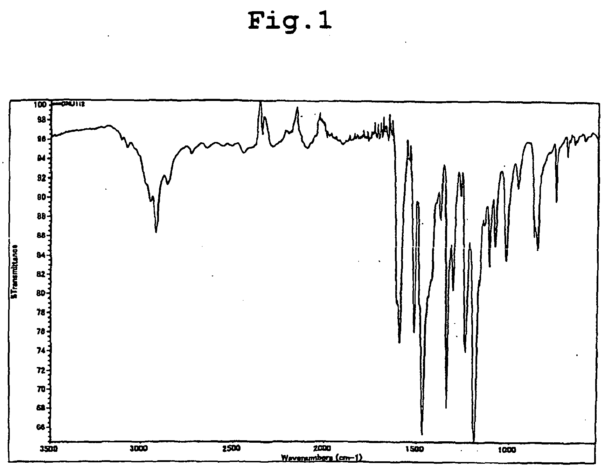

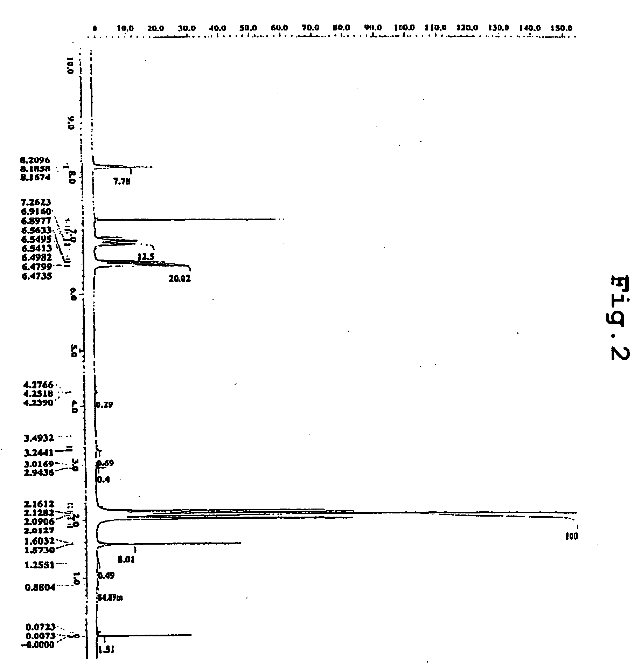

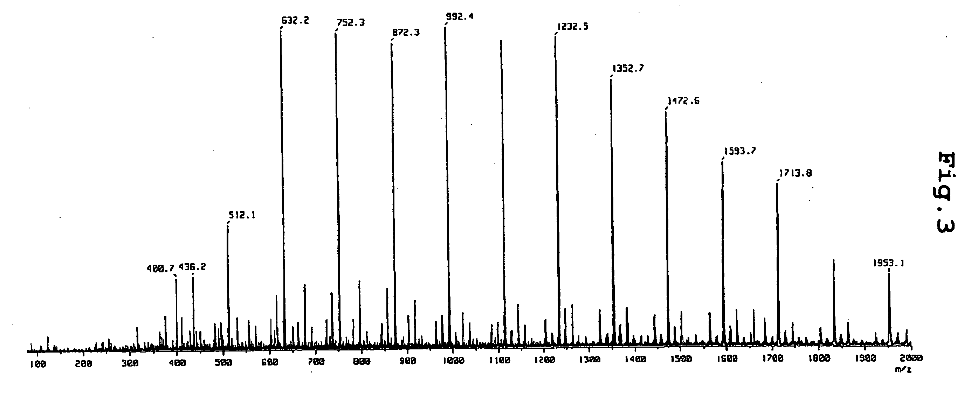

A 500-ml reactor having a stirrer, a reflux condenser, a thermometer and a Dean and Stark water separator was charged with 200.3 g of N,N-dimethylformamide, 70.4 g of the resin "A", 52.0 g (0.33 mol) of 4-chloronitrobenzene and 24.9 g (0.18 mol) of potassium carbonate. 19.1 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 291.9 g of methanol, to precipitate a solid. The solid was recovered by filtration, washed with methanol and then dried, to obtain 64.7 g of an aromatic dinitro compound (resin "G"). The resin "G" had a number average molecular weight of 1, 457 and a weight average molecular weight of 2,328. Fig. 1 shows an infrared absorption spectrum (IR) of the resin "G". Absorptions at a wavenumber of 1,520cm⁻¹ and a wavenumber of 1, 343 cm⁻¹, which correspond to an N-O bond, were found in the infrared absorption spectrum. Fig. 2 shows ¹H NMR spectrum of the resin "G". A peak corresponding to protons of a benzene ring where the protons were bonded to ortho positions of a nitro group was found around 8.2 ppm in the ¹H NMR spectrum. In regard to FD mass spectrum of the resin "G", an oligomer structure as shown in Fig. 3 was observed. This oligomer structure agrees with the theoretical molecular weight of the resin "G".

### Example 2

### (Synthesis of aromatic diamine compound)

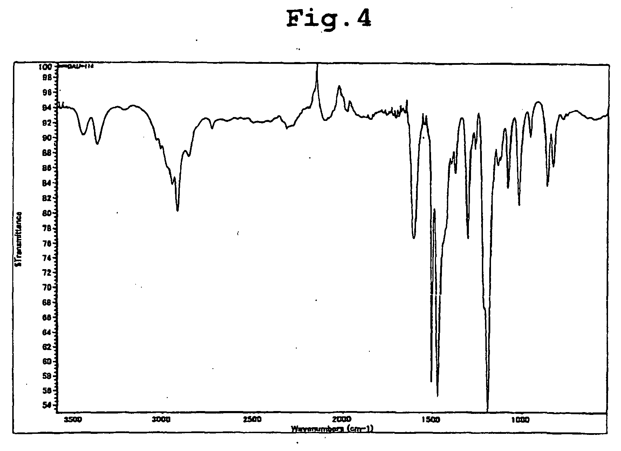

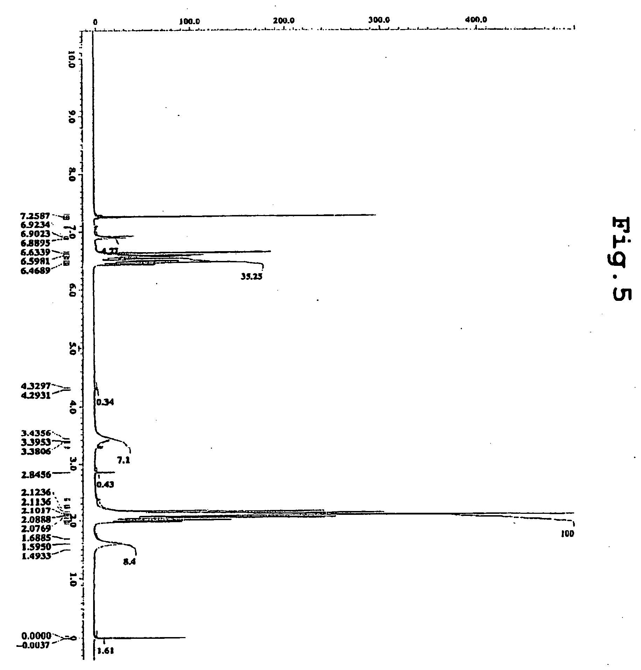

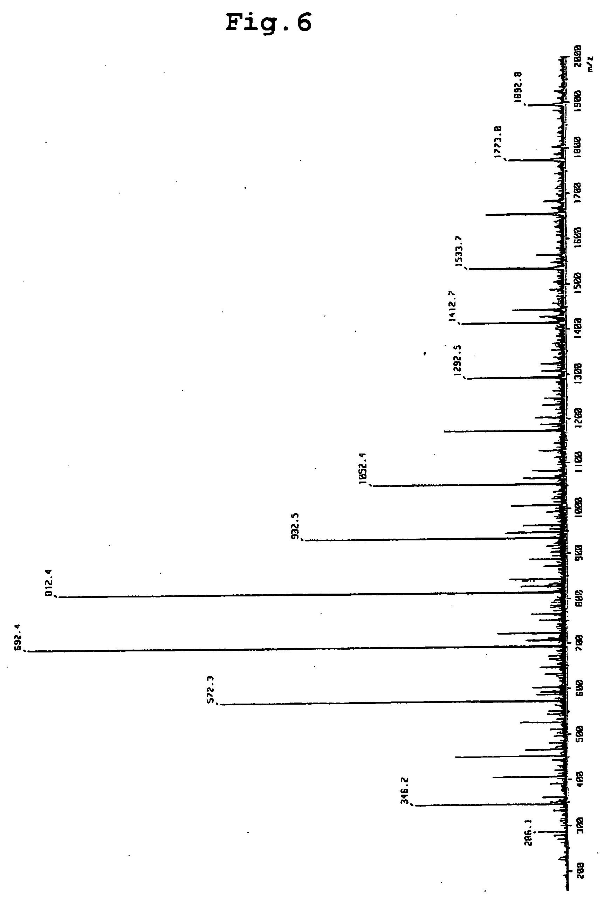

Then, a 100-ml reactor having a stirrer was charged with 1.16 g of the resin "G", 30.0 g of N,N-dimethylformamide and 167 mg of a 5%Pd/C catalyst. The mixture was vigorously stirred in a hydrogen atmosphere for 6 hours at room temperature, to allow the mixture react. Then, the reaction mixture was filtered to remove the catalyst, then concentrated with an evaporator and then dried under reduced pressure, to obtain 1.01 g of an aromatic diamine compound (resin "H"). The resin "H" had a number average molecular weight of 1,758 and a weight average molecular weight of 3,411. Fig. 4 shows an infrared absorption spectrum (IR) of the resin "H". Absorptions at a wavenumber of 3,448 cm⁻¹ and a wavenumber of 3,367 cm⁻¹, which correspond to an N-H bond, were found in the infrared absorption spectrum. Fig. 5 shows ¹H NMR spectrum of the resin "H". A peak of a proton corresponding to an amino group was found around 3.5 ppm in the ¹H NMR spectrum. In regard to FD mass spectrum of the resin "H", an oligomer structure as shown in Fig. 6 was observed. This oligomer structure agrees with the theoretical molecular weight of the resin "H".

### Example 3

### (Synthesis of aromatic dinitro compound)

A 500-ml reactor having a stirrer, a reflux condenser, a thermometer and a Dean and Stark water separator was charged with 250.2 g of N,N-dimethylformamide, 148.5 g of the resin "B", 52.1 g (0.33 mol) of 4-chloronitrobenzene and 25.0 g (0.18 mol) of potassium carbonate. 20.0 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 320.1 g of methanol, to precipitate a solid. The solid was recovered by filtration, washed with methanol and then dried, to obtain 140.3 g of an aromatic dinitro compound (resin "I"). The resin "I" had a number average molecular weight of 3,081 and a weight average molecular weight of 5,587. An infrared absorption spectrum (IR) of the resin "I" showed absorptions at a wavenumber of 1,519 cm⁻¹ and a wavenumber of 1,342 cm⁻¹, which correspond to an N-O bond.

### Example 4

### (Synthesis of aromatic diamine compound)

Then, a 100-ml reactor having a stirrer was charged with 1.20 g of the resin "I", 35.0 g of N,N-dimethylformamide and 156 mg of a 5%Pd/C catalyst. The mixture was vigorously stirred in a hydrogen atmosphere at room temperature for 8 hours, to allow the mixture react. Then, the reaction mixture was filtered to remove the catalyst, then concentrated with an evaporator and then dried under reduced pressure, to obtain 0.99 g of an aromatic diamine compound (resin "J"). The resin "J" had a number average molecular weight of 2,905 and a weight average molecular weight of 6,388. An infrared absorption spectrum (IR) of the resin "J" showed absorptions at a wavenumber of 3,447 cm⁻¹ and a wavenumber of 3,365 cm⁻¹, which correspond to an N-H bond.

### Example 5

### (Synthesis of aromatic dinitro compound)

A 500-ml reactor having a stirrer, a reflux condenser, a thermometer and a Dean and Stark water separator was charged with 200.2 g of N,N-dimethylformamide, 68.3 g of the resin "C", 52.2 g (0.33 mol) of 4-chloronitrobenzene and 24.9 g (0.18 mol) of potassium carbonate. 19.0 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 290.2 g of methanol, to precipitate a solid. The solid was recovered by filtration, washed with methanol and then dried, to obtain 63.8 g of an aromatic dinitro compound (resin "K"). The resin "K" had a number average molecular weight of 1,250 and a weight average molecular weight of 1,719. An infrared absorption spectrum (IR) of the resin "K" showed absorptions at a wavenumber of 1,522 cm⁻¹ and a wavenumber of 1,340 cm⁻¹, which correspond to an N-O bond.

### Example 6

### (Synthesis of aromatic diamine compound)

Then, a 100-ml reactor having a stirrer was charged with 1.15 g of the resin "K", 29.9 g of N,N-dimethylformamide and 160 mg of a 5%Pd/C catalyst. The mixture was vigorously stirred in a hydrogen atmosphere at room temperature for 6 hours, to allow the mixture react. Then, the reaction mixture was filtered to remove the catalyst, then concentrated with an evaporator and then dried under reduced pressure, to obtain 0.88 g of an aromatic diamine compound (resin "L"). The resin "L" had a number average molecular weight of 1,205 and a weight average molecular weight of 2,009. An infrared absorption spectrum (IR) of the resin "L" showed absorptions at a wavenumber of 3,446 cm⁻¹ and a wavenumber of 3,367 cm⁻¹, which correspond to an N-H bond.

### Example 7

### (Synthesis of aromatic dinitro compound)

A 500-ml reactor having a stirrer, a reflux condenser, a thermometer and a Dean and Stark water separator was charged with 250.5 g of N,N-dimethylformamide, 126.0 g of the resin "D", 51.9 g (0.33 mol) of 4-chloronitrobenzene and 25.0 g (0.18 mol) of potassium carbonate. 19.2 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 330.3 g of methanol, to precipitate a solid. The solid was recovered by filtration, washed with methanol and then dried, to obtain 115. 0 g of an aromatic dinitro compound (resin "M"). The resin "M" had a number average molecular weight of 2, 939 and a weight average molecular weight of 5, 982. An infrared absorption spectrum (IR) of the resin "M" showed absorptions at a wavenumber of 1, 518 cm⁻¹ and a wavenumber of 1,343 cm⁻¹, which correspond to an N-O bond.

### Example 8

### (Synthesis of aromatic diamine compound)

Then, a 100-ml reactor having a stirrer was charged with 2.13 g of the resin "M", 35.1 g of N,N-dimethylformamide and 189 mg of a 5%Pd/C catalyst. The mixture was vigorously stirred in a hydrogen atmosphere at room temperature for 8 hours, to allow the mixture react. Then, the reaction mixture was filtered to remove the catalyst, then concentrated with an evaporator and then dried under reduced pressure, to obtain 1.89 g of an aromatic diamine compound (resin "N"). The resin "N" had a number average molecular weight of 2,733 and a weight average molecular weight of 6,746. An infrared absorption spectrum (IR) of the resin "N" showed absorptions at a wavenumber of 3,449 cm⁻¹ and a wavenumber of 3,366 cm⁻¹, which correspond to an N-H bond.

### Example 9

### (Synthesis of aromatic dinitro compound)

A 500-ml reactor having a stirrer, a reflux condenser, a thermometer and a Dean and Stark water separator was charged with 200.1 g of N,N-dimethylformamide, 75.5 g of the resin "E", 52.0 g (0.33 mol) of 4-chloronitrobenzene and 25.0 g (0.18 mol) of potassium carbonate. 20.0 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 300.2 g of methanol, to precipitate a solid. The solid was recovered by filtration, washed with methanol and then dried, to obtain 72.1 g of an aromatic dinitro compound (resin "O"). The resin "O" had a number average molecular weight of 2, 103 and a weight average molecular weight of 5,194. An infrared absorption spectrum (IR) of the resin "O" showed absorptions at a wavenumber of 1,516 cm⁻¹ and a wavenumber of 1,340 cm⁻¹, which correspond to an N-O bond.

### Example 10

### (Synthesis of aromatic diamine compound)

Then, a 100-ml reactor having a stirrer was charged with 1.31 g of the resin "O", 30.0 g of N,N-dimethylformamide and 165 mg of a 5%Pd/C catalyst. The mixture was vigorously stirred in a hydrogen atmosphere at room temperature for 6 hours, to allow the mixture react. Then, the reaction mixture was filtered to remove the catalyst, then concentrated with an evaporator and then dried under reduced pressure, to obtain 1.10 g of an aromatic diamine compound (resin "P"). The resin "P" had a number average molecular weight of 2,051 and a weight average molecular weight of 6,142. An infrared absorption spectrum (IR) of the resin "P" showed absorptions at a wavenumber of 3,450 cm⁻¹ and a wavenumber of 3,365 cm⁻¹, which correspond to an N-H bond.

### Example 11

### (Synthesis of aromatic dinitro compound)

A 500-ml reactor having a stirrer, a reflux condenser, a thermometer and a Dean and Stark water separator was charged with 200.0 g of N,N-dimethylformamide, 70.7 g of the resin "F", 52.0 g (0.33 mol) of 4-chloronitrobenzene and 25.1 g (0.18 mol) of potassium carbonate. 19.3 g of toluene was added to the reactor and the atmosphere in the reactor was replaced with nitrogen. Then, the resultant mixture was heated and the mixture was continuously stirred for 5 hours at a temperature of 140 to 150°C, to allow the mixture to react. Water generated by the reaction was sequentially removed by azeotrope with toluene. After the completion of the reaction, filtration was carried out at 80 to 90°C, to remove an inorganic salt. Then, the thus-obtained filtrate was cooled down to room temperature. The filtrate was poured to 300.3 g of methanol, to precipitate a solid. The solid was recovered by filtration, washed with methanol and then dried, to obtain 64.1 g of an aromatic dinitro compound (resin "Q"). The resin "Q" had a number average molecular weight of 1,538 and a weight average molecular weight of 2,432. An infrared absorption spectrum (IR) of the resin "Q" showed absorptions at a wavenumber of 1, 522 cm⁻¹ and a wavenumber of 1,344 cm⁻¹, which correspond to an N-O bond.

### Example 12

### (Synthesis of aromatic diamine compound)

Then, a 100-ml reactor having a stirrer was charged with 1.50 g of the resin "Q", 30.0 g of N,N-dimethylformamide and 170 mg of a 5%Pd/C catalyst. The mixture was vigorously stirred in a hydrogen atmosphere at room temperature for 6 hours, to allow the mixture react. Then, the reaction mixture was filtered to remove the catalyst, then concentrated with an evaporator and then dried under reduced pressure, to obtain 1.14 g of an aromatic diamine compound (resin "R"). The resin "R" had a number average molecular weight of 1,465 and a weight average molecular weight of 2,809. An infrared absorption spectrum (IR) of the resin "R" showed absorptions at a wavenumber of 3,447 cm⁻¹ and a wavenumber of 3, 360 cm⁻¹, which correspond to an N-H bond.

## Claims

1. An aromatic diamine compound represented by the formula (1), 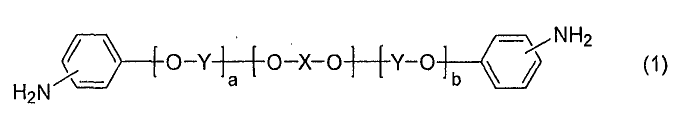 wherein -(O-X-O)- represents a moiety of the formula (2) or the formula (3), -(Y-O)- represents an arrangement of a moiety of the formula (4) or a random arrangement of at least two kinds of moieties of the formula (4), each of a and b is an integer of 0 to 100, provided that at least one of a and b is not 0, and each amino group is substituted at a para position or a meta position, 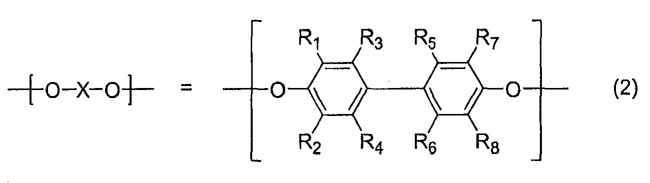 wherein R₁, R₂, R₃, R₇ and R₈ are the same or different and represent a halogen atom, an alkyl group having 6 or less carbon atoms or a phenyl group and R₄, R₅ and R₆ are the same or different and represent a hydrogen atom, a halogen atom, an alkyl group having 6 or less carbon atoms or a phenyl group, 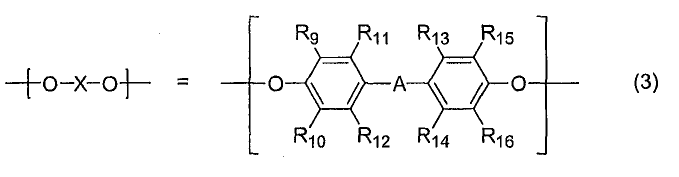 wherein R₉, R₁₀, R₁₁, R₁₂, R₁₃, R₁₄, R₁₅ and R₁₆ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 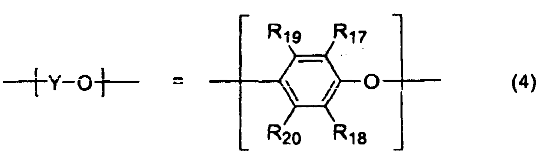 wherein R₁₇ and R₁₈ are the same or different and represent a halogen atom, an alkyl group having 6 or less carbon atoms or a phenyl group and R₁₉ and R₂₀ are the same or different and represent a hydrogen atom, a halogen atom, an alkyl group having 6 or less carbon atoms or a phenyl group.

2. The aromatic diamine compound according to claim 1, wherein - (O-X-O) - is a moiety of the formula (5), the formula (6) or the formula (7) and -(Y-O)- represents an arrangement of a moiety of the formula (8) or the formula (9) or a random arrangement of moieties of the formula (8) and the formula (9), 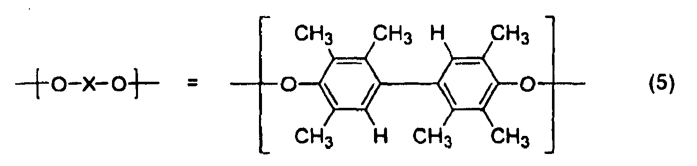 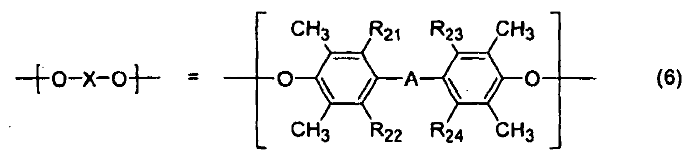 wherein R₂₁, R₂₂, R₂₃ and R₂₄ are the same or different and represent a hydrogen atom or a methyl group and -A-represents a linear, branched or cyclic bivalent hydrocarbon group having 20 or less carbon atoms, 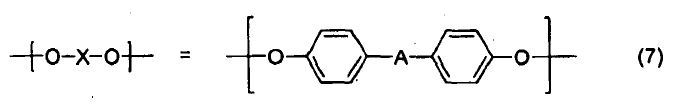 wherein -A- represents a linear, branched or cyclic bivalent hydrocarbon group having 20 or less carbon atoms.

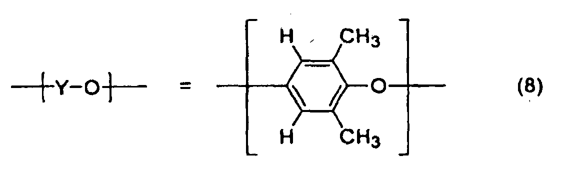

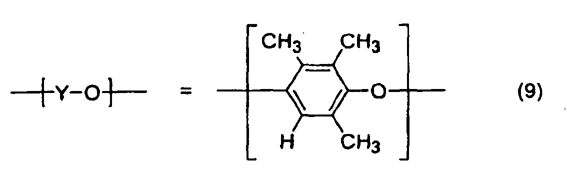

3. The aromatic diamine compound according to claim 1, wherein the aromatic diamine compound has a number average molecular weight of 500 to 3,000.

4. A process for the production of the aromatic diamine compound as defined in claim 1, comprising reducing an aromatic dinitro compound represented by the formula (10), 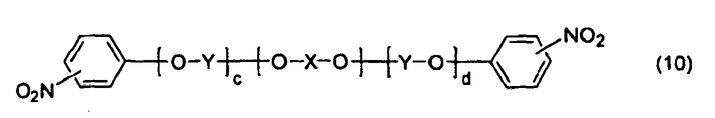 wherein -(O-X-O)- represents a moiety of the formula (11) or the formula (12), -(Y-O)- represents an arrangement of a moiety of the formula (13) or a random arrangement of at least two kinds of moieties of the formula (13), each of c and d is an integer of 0 to 100, provided that at least one of c and d is not 0, and each nitro group is substituted at a para position or a meta position, 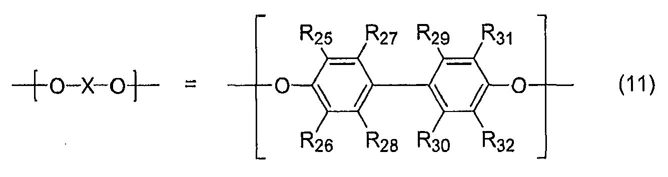 wherein R₂₅, R₂₆, R₂₇, R₃₁ and R₃₂ are the same or different and represent a halogen atom, an alkyl group having 6 or less carbon atoms or a phenyl group, R₂₈, R₂₉ and R₃₀ are the same or different and represent a hydrogen atom, a halogen atom, an alkyl group having 6 or less carbon atoms or a phenyl group, 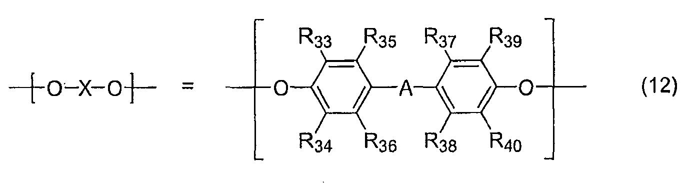 wherein R₃₃, R₃₄, R₃₅, R₃₆, R₃₇, R₃₈, R₃₉ and R₄₀ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 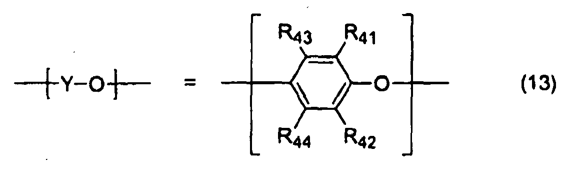 wherein R₄₁ and R₄₂ are the same or different and represent a halogen atom, an alkyl group having 6 or less carbon atoms or a phenyl group and R₄₃ and R₄₄ are the same or different and represent a hydrogen atom, a halogen atom, an alkyl group having 6 or less carbon atoms or a phenyl group.

5. An aromatic dinitro compound, which is the aromatic dinitro compound of the formula (10) as defined in claim 4.

6. The aromatic dinitro compound according to claim 5, wherein - (O-X-O) - is a moiety of the formula (14), the formula (15) or the formula (16) and -(Y-O)- represents an arrangement of a moiety of the formula (17) or the formula (18) or a random arrangement of moieties of the formula (17) and the formula (18), 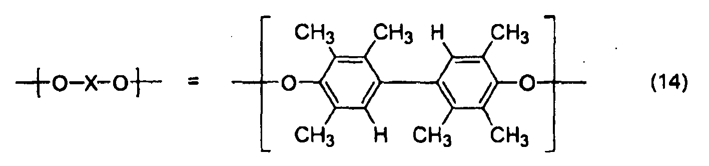 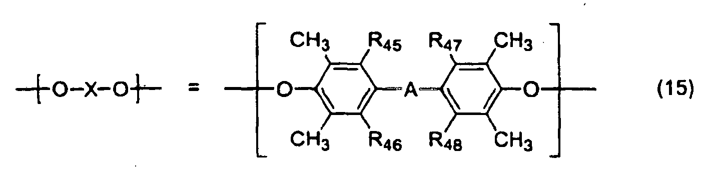 wherein R₄₅, R₄₆, R₄₇ and R₄₈ are the same or different and represent a hydrogen atom or a methyl group and -A-represents a linear, branched or cyclic bivalent hydrocarbon group having 20 or less carbon atoms, 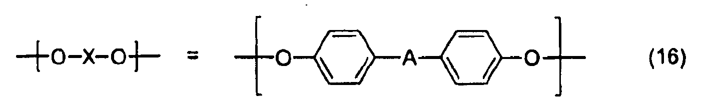 wherein -A- represents a linear, branched or cyclic bivalent hydrocarbon group having 20 or less carbon atoms.

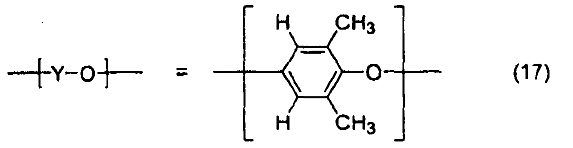

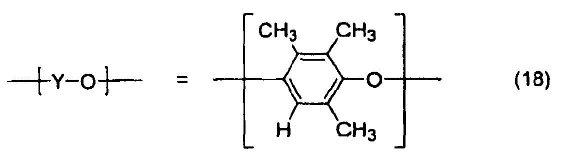

7. The aromatic dinitro compound according to claim 5, wherein the aromatic dinitro compound has a number average molecular weight of 500 to 3,000.

8. A process for the production of the aromatic dinitro compound as defined in claim 5, comprising reacting a bifunctional phenylene ether oligomer obtained by oxidative coupling of a bifunctional phenol compound represented by the formula (19) or (20) and a monofunctional phenol compound represented by the formula (21) with a nitro halobenzene compound or a dinitro benzene compound, 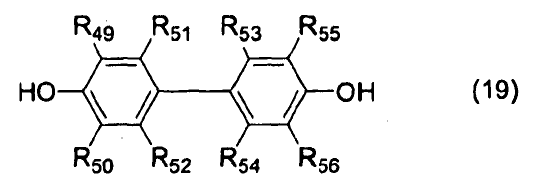 wherein R₄₉, R₅₀, R₅₁, R₅₅ and R₅₆ are the same or different and represent a halogen atom, an alkyl group having 6 or less carbon atoms or a phenyl group and R₅₂, R₅₃ and R₅₄ are the same or different and represent a hydrogen atom, a halogen atom, an alkyl group having 6 or less carbon atoms or a phenyl group, 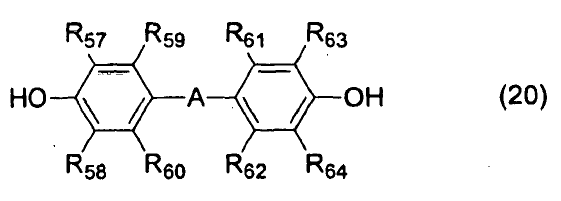 wherein R₅₇, R₅₈, R₅₉, R₆₀, R₆₁, R₆₂, R₆₃ and R₆₄ are the same or different and represent a hydrogen atom, a halogen atom, an alkyl group having 6 or less carbon atoms or a phenyl group and -A- represents a linear, branched or cyclic bivalent hydrocarbon group having 20 or less carbon atoms, 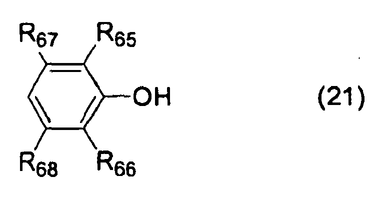 wherein R₆₅ and R₆₆ are the same or different and represent a halogen atom, an alkyl group having 6 or less carbon atoms or a phenyl group and R₆₇ and R₆₈ are the same or different and represent a hydrogen atom, a halogen atom, an alkyl group having 6 or less carbon atoms or a phenyl group.

## Patentansprüche

1. Aromatische Diaminverbindung, dargestellt durch die Formel (1), 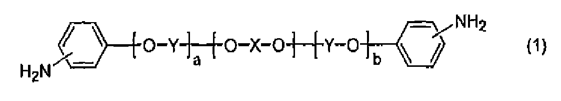 wobei-(O-X-O)- eine Gruppe der Formel (2) oder der Formel (3) darstellt, -(Y-O)- eine Anordnung einer Gruppe der Formel (4) oder eine Zufallsanordnung von wenigstens zwei Arten von Gruppen der Formel (4) darstellt, wobei jedes von a und b eine ganze Zahl von 0 bis 100 ist, unter der Voraussetzung, dass wenigstens eines von a und b nicht 0 ist, und wobei jede Aminogruppe an einer para-Position oder meta-Position substituiert ist, 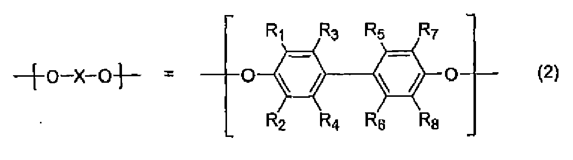 wobei R₁, R₂, R₃, R₇ und R₈ dieselben oder unterschiedlich sind und ein Halogenatom, eine Alkylgruppe mit 6 oder weniger Kohlenstoffatomen oder eine Phenylgruppe darstellen, und wobei R₄, R₅ und R₆ dieselben oder unterschiedlich sind und ein Wasserstoffatom, ein Halogenatom, eine Alkylgruppe mit 6 oder weniger Kohlenstoffatomen oder eine Phenylgruppe darstellen, 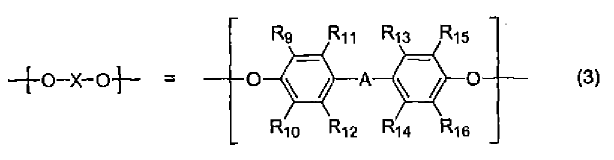 wobei R₉, R₁₀, R₁₁, R₁₂, R₁₃, R₁₄, R₁₅ und R₁₆ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen darstellt, 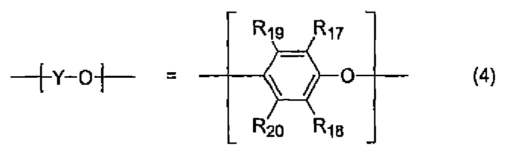 wobei R₁₇ und R₁₈ dieselben oder unterschiedlich sind und ein Halogenatom, eine Alkylgruppe mit 6 oder weniger Kohlenstoffatomen oder ein Phenylgruppe darstellen, und wobei R₁₉ und R₂₀ dieselben oder unterschiedlich sind und ein Wasserstoffatom, ein Halogenatom, eine Alkylgruppe mit 6 oder weniger Kohlenstoffatomen oder eine Phenylgruppe darstellen.

2. Aromatische Diaminverbindung nach Anspruch 1, wobei -(O-X-O)- eine Gruppe der Formel (5), der Formel (6) oder der Formel (7) ist, und wobei -(Y-O)- eine Anordnung einer Gruppe der Formel (8) oder der Formel (9) oder eine Zufallsanordnung von Gruppen der Formel (8) und der Formel (9) darstellt, 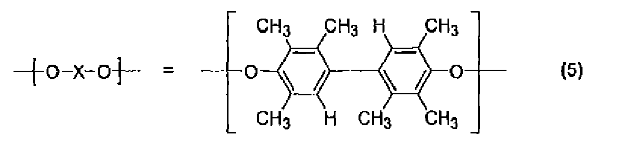 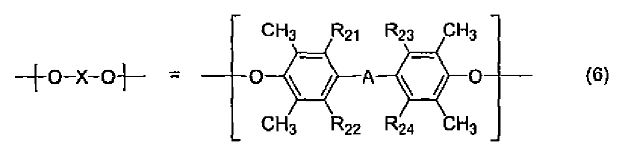 wobei R₂₁, R₂₂, R₂₃ und R₂₄ dieselben oder unterschiedlich sind und ein Wasserstoffatom oder ein Methylgruppe darstellen und wobei -A- eine lineare, verzweigte oder zyklische bivalente Kohlenwasserstoffgruppe mit 20 oder weniger Kohlenstoffatomen darstellt, 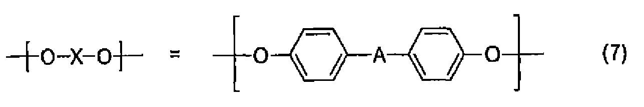 wobei -A- eine lineare, verzweigte oder zyklische bivalente Kohlenwasserstoffgruppe mit 20 oder weniger Kohlenstoffatomen darstellt

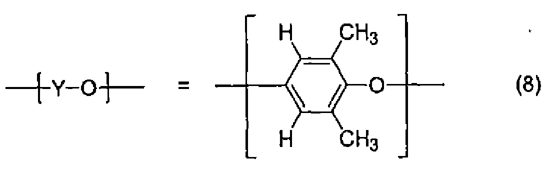

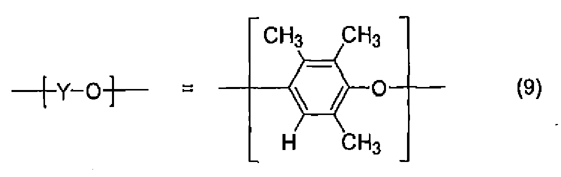

3. Aromatische Diaminverbindungen nach Anspruch 1, wobei die aromatische Diaminverbindung eine zahlengennittelte Molmasse (number average molecular weight) von 500 bis 3.000 hat.

4. Verfahren zur Herstellung der aromatischen Diaminverbindung, wie in Anspruch 1 definiert, umfassend die Reduktion einer aromatischen Dinitroverbindung, dargestellt durch die Formel (10), 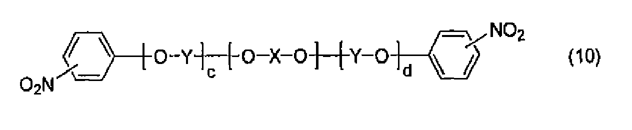 wobei -(O-X-O)- eine Gruppe der Formel (11) oder der Formel (12) darstellt, -(Y-O)-eine Anordnung einer Gruppe der Formel (13) oder eine Zufallsanordnung von wenigstens zwei Arten von Gruppen der Formel (13) darstellt, wobei jedes von c und d eine ganze Zahl von 0 bis 100 ist, unter der Voraussetzung, dass wenigstens eines von c und d nicht 0 ist, und wobei jede Nitrogruppe an einer para-Position oder einer meta-Position substituiert ist, 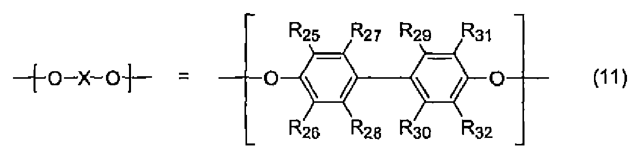 wobei R₂₅, R₂₆, R₂₇, R₃₁ und R₃₂ dieselben oder unterschiedlich sind und ein Halogenatom, eine Alkylgruppe mit 6 oder weniger Kohlenstoffatomen oder eine Phenylgruppe darstellen, R₂₈, R₂₉ und R₃₀ dieselben oder unterschiedlich sind und ein Wasserstoffatom, ein Halogenatom, eine Alkylgruppe mit 6 oder weniger Kohlenstoffatomen oder eine Phenylgruppe darstellen, 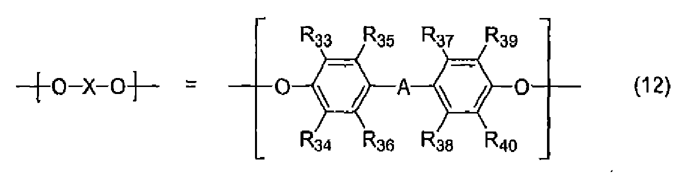 wobei R₃₃, R₃₄, R₃₅, R₃₆, R₃₇, R₃₈, R₃₉ und R₄₀ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen darstellt, 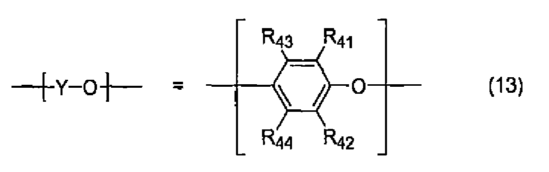 wobei R₄₁ und R₄₂ dieselben oder unterschiedlich sind und ein Halogenatom, eine Alkylgruppe mit 6 oder weniger Kohlenstoffatomen oder eine Phenylgruppe darstellen, und wobei R₄₃ und R₄₄ dieselben oder unterschiedlich sind und ein Wasserstoffatom, ein Halogenatorn, eine Alkylgruppe mit 6 oder weniger Kohlenstoffatomen oder eine Phenylgruppe darstellen.

5. Aromatische Dinitroverbindung, die die aromatische Dinitroverbindung der Formel (10) ist, wie in Anspruch 4 definiert.

6. Aromatische Dinitroverbindung nach Anspruch 5, wobei -(O-X-O)- eine Gruppe der Formel (14), der Formel (15) oder der Formel (16) ist, und wobei -(Y-O)- eine Anordnung einer Gruppe der Formel (17) oder der Formel (18) oder eine Zufallsanordnung von Gruppen der Formel (17) und der Formel (18) ist, 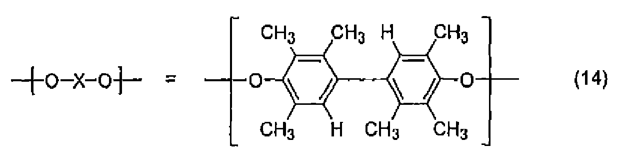 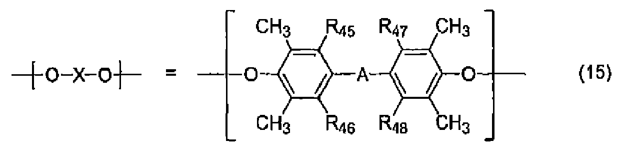 wobei R₄₅, R₄₆, R₄₇ und R₄₈ dieselben oder unterschiedlich sind und ein Wasserstoffatom oder ein Methylgruppe darstellen, und wobei -A- eine lineare, verzweigte oder zyklische bivalente Kohlenwasserstoffgruppe mit 20 oder weniger Kohlenstoffatomen darstellt, 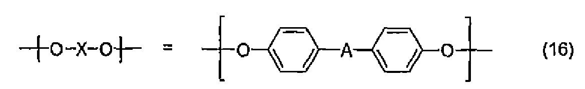 wobei -A- eine lineare, verzweigte oder zyklische bivalente Kohlenwasserstoffgruppe mit 20 oder weniger Kohlenstoffatomen darstellt,

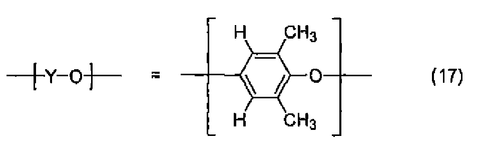

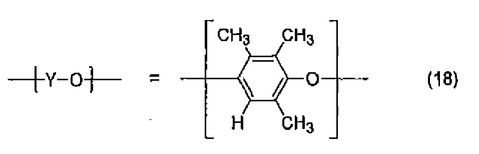

7. Aromatische Dinitroverbindungen nach Anspruch 5, wobei die aromatische Dinitroverbindung eine zahlengemittelte Molmasse von 500 bis 3.000 hat.

8. Verfahren zur Herstellung der aromatischen Dinitroverhindung, wie in Anspruch 5 definiert, umfassend das Umsetzen eines bifunktionalen Phenylenetheroligomers, erhalten durch oxidative Kopplung einer bifunktionalen Phenolverbindung, dargestellt durch die Formel (19) oder (20), und einer monofunktionalen Phenolverbindung, dargestellt durch die Formel (21), mit einer Nitrohalogenbenzolverbindung oder einer Dinitro-benzolverbindung, 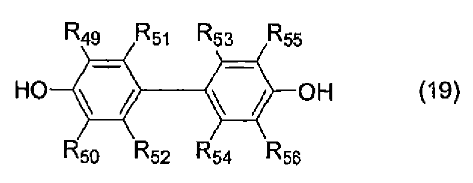 wobei R₄₉, R₅₀, R₅₁, R₅₅ und R₅₆ dieselben oder unterschiedlich sind und ein Halogenatom, eine Alkylgruppe mit 6 oder weniger Kohlenstoffatomen oder eine Phenylgruppe darstellen, und wobei R₅₂, R₅₃ und R₅₄ dieselben oder unterschiedlich sind und ein Wasserstoffatom, ein Halogenatom, eine Alkylgruppe mit 6 oder weniger Kohlenstoffatomen oder eine Phenylgruppe darstellen, 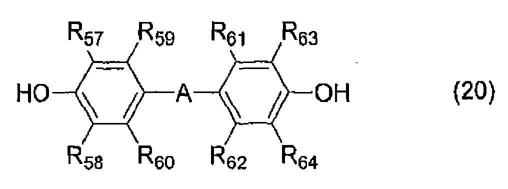 wobei R₅₇, R₅₈, R₅₉, R₆₀, R₆₁, R₆₂, R₆₃ und R₆₄ dieselben oder unterschiedlich sind und ein Wasserstoffatom, ein Halogenatom, eine Alkylgruppe mit 6 oder weniger Kohlenstoffatomen oder eine Phenylgruppe darstellen, und wobei -A- eine lineare, verzweigte oder zyklische bivalente Kohlenwasserstoffgruppe mit 20 oder weniger Kohlenstoffatomen darstellt, 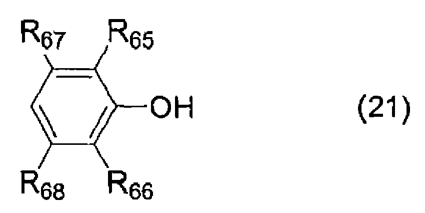 wobei R₆₅ und R₆₆ dieselben oder unterschiedlich sind und ein Halogenatom, eine Alkylgruppe mit 6 oder weniger Kohlenstoffatomen oder eine Phenylgruppe darstellen, und wobei R₆₇ und R₆₈ dieselben oder unterschiedlich sind und ein Wasserstoffatom, ein Halogenatom, eine Alkylgruppe mit 6 oder weniger Kohlenstoffatomen oder eine Phenylgruppe darstellen.

## Revendications

1. Composé diamine aromatique représenté par la formule (1), 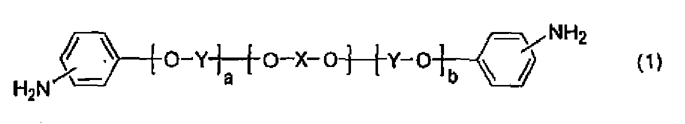 dans laquelle -(O-X-O)- représente un fragment de formule (2) ou de formule (3), -(Y-O)- représente un arrangement d'un fragment de formule (4) ou un arrangement statistique d'au moins deux types de fragments de formule (4), chacun de a et b est un nombre entier de 0 à 100, à condition qu'au moins un de a et b n'est pas 0, et chaque groupe amino est substitué en une position para ou une position méta, 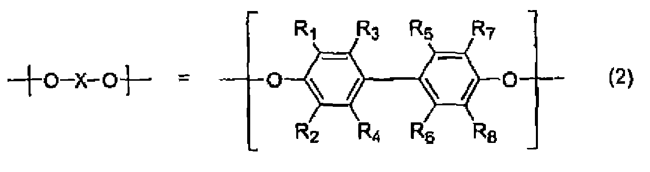 dans laquelle R₁, R₂, R₃, R₇ et R₈ sont identiques ou différents et représentent un atome d'halogène, un groupe alkyle ayant 6 atomes de carbone ou moins ou un groupe phényle et R₄, R₅ et R₆ sont identiques ou différents et représentent un atome d'hydrogène, un atome d'halogène, un groupe alkyle ayant 6 atomes de carbone ou moins ou un groupe phényle, 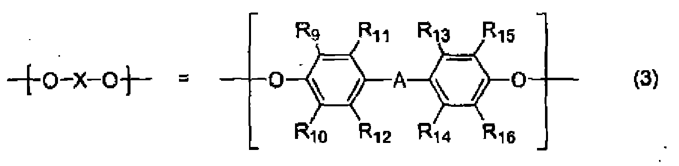 dans laquelle R₉, R₁₀, R₁₁, R₁₂, R₁₃, R₁₄, R₁₅ et R₁₆ sont identiques ou différents et représentent un atome d'hydrogène, un atome d'halogène, un groupe alkyle ayant 6 atomes de carbone ou moins ou un groupe phényle et -A- représente un groupe hydrocarboné bivalent linéaire, ramifié ou cyclique ayant 20 atomes de carbone ou moins, 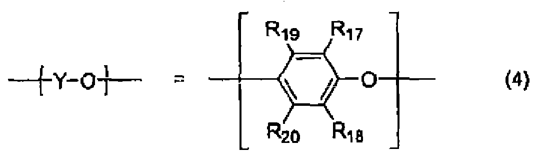 dans laquelle R₁₇ et R₁₈ sont identiques ou différents et représentent un atome d'halogène, un groupe alkyle ayant 6 atomes de carbone ou moins ou un groupe phényle et R₁₉ et R₂₀ sont identiques ou différents et représentent un atome d'hydrogène, un atome d'halogène, un groupe alkyle ayant 6 atomes de carbone ou moins ou un groupe phényle.

2. Composé diamine aromatique selon la revendication 1, dans lequel -(O-X-O)- est un fragment de formule (5), de formule (6) ou de formule (7) et -(Y-O)- représente un arrangement d'un fragment de formule (8) ou de formule (9) ou un arrangement statistique de fragments de formule (8) et de formule (9), 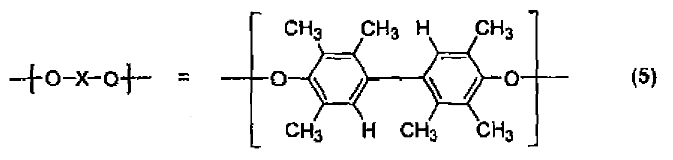 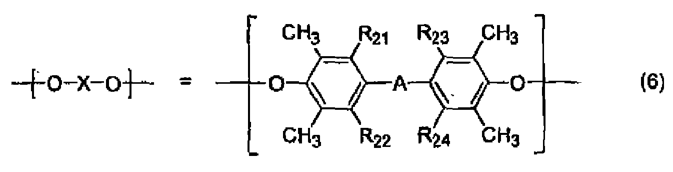 dans lesquelles R₂₁, R₂₂, R₂₃ et R₂₄ sont identiques ou différents et représentent un atome d'hydrogène ou un groupe méthyle et -A- représente un groupe hydrocarboné bivalent linéaire, ramifié ou cyclique ayant 20 atomes de carbone ou moins, 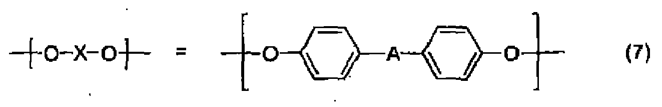 dans laquelle -A- représente un groupe hydrocarboné bivalent linéaire, ramifié ou cyclique ayant 20 atomes de carbone ou moins,

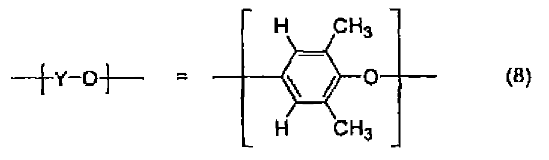

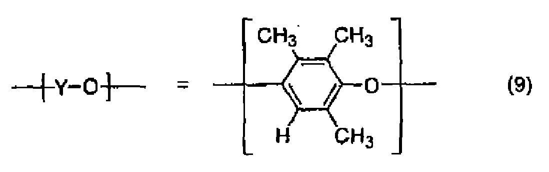

3. Composé diamine aromatique selon la revendication 1, dans lequel le composé diamine aromatique a un poids moléculaire moyen en nombre de 500 à 3000.

4. Procédé de production du composé diamine aromatique tel que défini selon la revendication 1, comprenant la réduction d'un composé dinitro aromatique représenté par la formule (10), 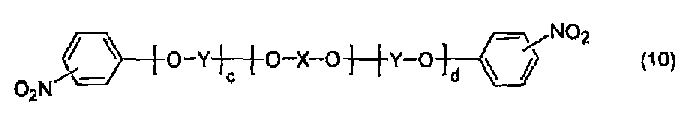 dans laquelle -(O-X-O)- représente un fragment de formule (11) ou de formule (12), -(Y-O)- représente un arrangement d'un fragment de formule (13) ou un arrangement statistique d'au moins deux types de fragments de formule (13), chacun de c et d est un nombre entier de 0 à 100, à condition qu'au moins un de c et d ne soit pas 0, et chaque groupe nitro est substitué en position para ou en position méta, 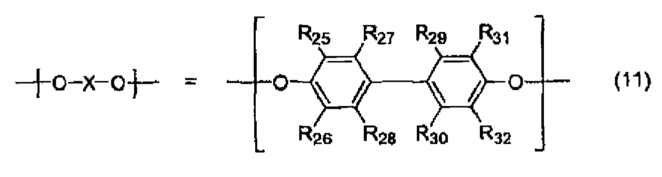 dans laquelle R₂₅, R₂₆, R₂₇, R₃₁ et R₃₂ sont identiques ou différents et représentent un atome d'halogène, un groupe alkyle ayant 6 atomes de carbone ou moins ou un groupe phényle, R₂₈, R₂₉ et R₃₀ sont identiques ou différents et représentent un atome d'hydrogène, un atome d'halogène, un groupe alkyle ayant 6 atomes de carbone ou moins ou un groupe phényle, 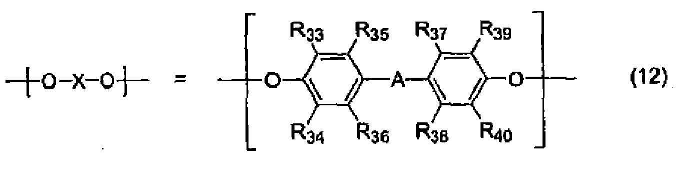 dans laquelle R₃₃, R₃₄, R₃₅, R₃₆, R₃₇, R₃₈, R₃₉ et R₄₀ sont identiques ou différents et représentent un atome d'hydrogène, un atome d'halogène, un groupe alkyle ayant 6 atomes de carbone ou moins ou un groupe phényle et -A- représente un groupe hydrocarboné bivalent linéaire, ramifié ou cyclique ayant 20 atomes de carbone ou moins, 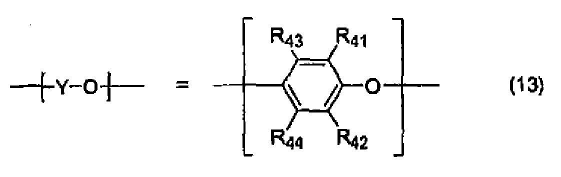 dans laquelle R₄₁ et R₄₂ sont identiques ou différents et représentent un atome d'halogène, un groupe alkyle ayant 6 atomes de carbone ou moins ou un groupe phényle et R₄₃ et R₄₄ sont identiques ou différents et représentent un atome d'hydrogène, un atome d'halogène, un groupe alkyle ayant 6 atomes de carbone ou moins ou un groupe phényle.

5. Composé dinitro aromatique, qui est le composé dinitro aromatique de formule (10) tel que défini selon la revendication 4.

6. Composé dinitro aromatique selon la revendication 5, dans lequel -(O-X-O)- est un fragment de formule (14), de formule (15) ou de formule (16) et -(Y-O)- représente un arrangement d'un fragment de formule (17) ou de formule (18) ou un arrangement statistique de fragments de formule (17) et de formule (18), 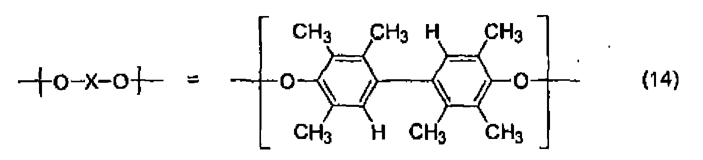 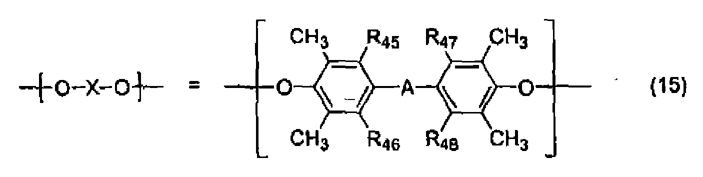 dans lesquelles R₄₅, R₄₆, R₄₇ et R₄₈ sont identiques ou différents et représentent un atome d'hydrogène ou un groupe méthyle et -A- représente un groupe hydrocarboné bivalent linéaire, ramifié ou cyclique ayant 20 atomes de carbone ou moins, 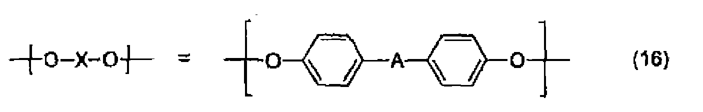 dans laquelle -A- représente un groupe hydrocarboné bivalent linéaire, ramifié ou cyclique ayant 20 atomes de carbone ou moins,

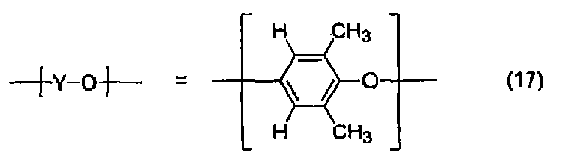

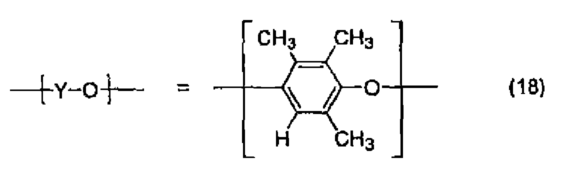

7. Composé dinitro aromatique selon la revendication 5, dans lequel le composé diamine aromatique a un poids moléculaire moyen en nombre de 500 à 3000.

8. Procédé de production du composé dinitro aromatique tel que défini selon la revendication 5, comprenant la mise en réaction d'un oligomère d'éther de phénylène bifonctionnel obtenu par couplage oxydatif d'un composé phénolique bifonctionnel représenté par la formule (19) ou (20) et un composé phénolique monofonctionnel représenté par la formule (21) avec un composé nitrohalogénobenzène ou un composé dinitrobenzène, 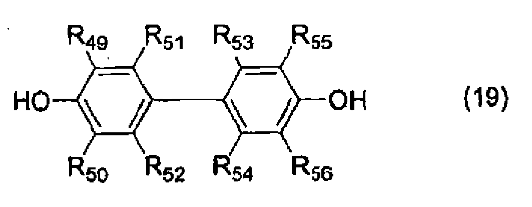 dans laquelle R₄₉, R₅₀, R₅₁, R₅₅ et R₅₆ sont identiques ou différents et représentent un atome d'halogène, un groupe alkyle ayant 6 atomes de carbone ou moins ou un groupe phényle et R₅₂, R₅₃ et R₅₄ sont identiques ou différents et représentent un atome d'hydrogène, un atome d'halogène, un groupe alkyle ayant 6 atomes de carbone ou moins ou un groupe phényle, 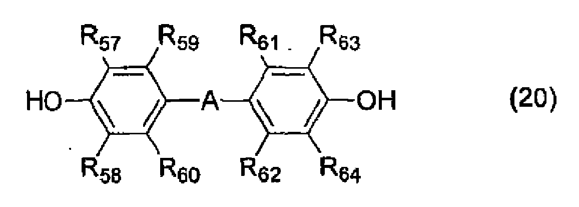 dans laquelle R₅₇, R₅₈, R₅₉, R₆₀, R₆₁, R₆₂, R₆₃ et R₆₄ sont identiques ou différents et représentent un atome d'hydrogène, un atome d'halogène, un groupe alkyle ayant 6 atomes de carbone ou moins ou un groupe phényle et -A- représente un groupe hydrocarboné bivalent linéaire, ramifié ou cyclique ayant 20 atomes de carbone ou moins, 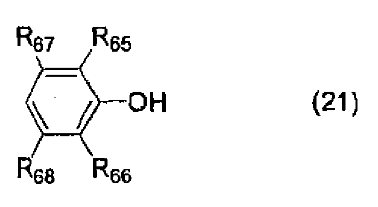 dans laquelle R₆₅ et R₆₆ sont identiques ou différents et représentent un atome d'halogène, un groupe alkyle ayant 6 atomes de carbone ou moins ou un groupe phényle et R₆₇ et R₆₈ sont identiques ou différents et représentent un atome d'hydrogène, un atome d' ha.logène, un groupe alkyle ayant 6 atomes de carbone ou moins ou un groupe phényle.
